# EUROPEAN PATENT SPECIFICATION

(11) **EP 0 368 857 B1**
(45) Date of publication and mention of the grant of the patent: **06.08.1997**
(21) Application number: 88903676.0
(22) Date of filing: 31.03.1988
(51) Int. Cl.: C07K 1/107, C12P 21/02, C07K 14/00

(54) **PROCESS FOR RECOVERING PURIFIED, OXIDIZED, RENATURED RECOMBINANT INTERLEUKIN-2 FROM MICROORGANISMS**
PROZESS ZUR GEWINNUNG VON GEREINIGTEM, OXIDIERTEM, RENATURIERTEM, REKOMBINANTEM INTERLEUKIN-2 AUS MIKROORGANISMEN
PROCEDE DE RECUPERATION, A PARTIR DE MICROORGANISMES, D'INTERLEUKINE-2 RECOMBINANTE, PURIFIEE, OXYDEE ET RENATUREE

(30) Priority: 11.05.1987 US 48408; 25.03.1988 US 167144
(43) Date of publication of application: 23.05.1990
(73) Proprietor: CHIRON CORPORATION, Emeryville, California 94608 (US)
(72) Inventor: WOLFE, Sidney, N., El Cerrito, CA 94530 (US); DORIN, Glenn, San Rafael, CA 94901 (US); DAVIS, John, T., Berkeley, CA 94703 (US); SMITH, Flint, Oakland, CA 94610 (US); LIM, Amy, San Francisco, CA 94116 (US)
(74) Representative: Bizley, Richard Edward
(86) International application number: US8801043
(87) International publication number: WO8808849

(56) References cited:
- EP-A- 0 114 506
- EP-A- 0 156 354
- EP-A- 0 185 459
- EP-A- 0 206 828
- Methods in Enzymology, volume 131, 1986, Academic Press Inc., R. Jaenicke et al.: "Refolding and association of oligomeric proteins", pages 218-250

## Description

### Technical Field

The invention is in the field of biochemistry and relates to a process for recovering purified, renatured recombinant interleukin-2 (IL-2) from the microorganisms in which it is produced.

### Background

IL-2, a lymphokine which is produced by normal peripheral blood lymphocytes and induces proliferation of antigen or mitogen stimulated T cells after exposure to plant lectins, antigens, or other stimuli, was first described by Morgan, D.A., et al, Science (1976) 193:1007-1008. Then called T cell growth factor because of its ability to induce proliferation of stimulated T lymphocytes, it is now recognized that in addition to its growth factor properties it modulates a variety of functions of immune system cells in vitro and in vivo and has been renamed IL-2. IL-2 is one of several lymphocyte-produced messenger-regulatory molecules that mediate immunocyte interactions and functions.

IL-2 was initially made by cultivating human peripheral blood lymphocytes (PBL) or other IL-2-producing cell lines. See, for instance, U.S. Patent No. 4,401,756. Recombinant DNA technology has provided an alternative to PBLs and cell lines for producing IL-2. Taniguchi, T., et al, Nature (1983) 302:305-310 and Devos, R., Nucleic Acids Research (1983) 11:4307-4323 have reported cloning the human IL-2 gene and expressing it in microorganisms.

Native human IL-2 is an antigen-nonspecific, genetically unrestricted soluble factor produced by erythrocyte rosette positive T cells stimulated with antigens, mitogens or alloantigens. It is a protein with a reported molecular weight in the approximate range of 13,000 to 17,000 daltons (S. Gillis and J. Watson, J Exp Med (1980) 159:1709) and an isoelectric point in the approximate range of pH 6-8.5. Human IL-2 has a number of in vitro and in vivo effects including enhancing the proliferative responses of human peripheral blood mononuclear cells or murine thymocytes, enhancing the immune response in humans and in animals against bacterial, parasitic, fungal, protozoan and viral infections, and supporting the growth of continuous T cell lines.

Human IL-2 has been obtained from genetically engineered E. coli as an unglycosylated protein with biological activities equivalent to those of native, glycosylated IL-2. (Taniguchi et al, Nature (1983) 11:4307-4323; Rosenberg et al, Science (1984) 223:1412-1415; Wang et al, Science (1984) 224:1431-1433; and Doyle et al, J Biol Resp Modifiers (1985) 4:96-109). Rosenberg and his coworkers have shown that systemic administration of recombinant IL-2 in high doses causes regression of established metastatic cancers in mice (Rosenberg et al, J Exp Med (1985) 161:1169-1188); and, in conjunction with lymphokine-activated killer cells (Rosenberg et al, New Eng J Med (1985) 313:1485-1492) and tumor-infiltration lymphocytes (Rosenberg et al, Science (1986) 233:1318-1321), in humans.

U.S. Patent No. 4,518,584 discloses muteins (analogs) of IL-2 in which the cysteine normally occurring at position 125 of the wild-type or native molecule has been replaced with a neutral amino acid, such as serine or alanine. European Patent (EP) publication 200,280 discloses muteins of IL-2 whereby the methionine at position 104 has been replaced by a conservative amino acid.

Microbially produced IL-2 is not glycosylated and is primarily produced in a denatured state. It is largely insoluble and, when expressed at high levels, it precipitates intracellularly in the form of "refractile" or "inclusion" bodies which appear as bright spots visible within the enclosure of the cell under a phase contrast microscope at magnifications down to 1000 fold. The problem addressed by the present invention is how to efficiently recover the IL-2 from the cell in a purified, cystine-bridged, renatured form that is acceptable for clinical use.

The heretofore available methods for recovering microbially produced IL-2 are described below.

U.S. Patent No. 4,569,790 describes a process for recovering recombinant IL-2 from an IL-2-producing microorganism in which the cell is disrupted, the disruptate is extracted with an aqueous solution of a chaotropic agent such as urea, the IL-2 is solubilized with a surfactant, e.g., sodium dodecyl sulfate (SDS), and the IL-2 is separated in the presence of a reducing agent.

Commonly owned U.S. Patents Nos. 4,530,787 and 4,572,978 described processes for purifying recombinant IL-2 from microorganisms in which partially purified reduced IL-2 is selectively oxidized under controlled conditions to its oxidized (cystine) form. The former patent uses o-iodosobenzoic acid as an oxidizing agent and the latter uses Cu⁺² cation as an oxidation promoter.

European Patent publication 206,828 published 30 December 1986, and entitled "Process for Recovering Refractile Bodies Containing Heterologous Proteins from Microbial Hosts" disclose methods for recovering and purifying refractile bodies of IL-2 from E. coli. To isolate the refractile material, the processes initially involve disrupting the cell wall and membrane of the host cell, removing greater than 99% by weight of the salts from the disruptate, redisrupting the desalted disruptate, adding a material to the disruptate to create a density or viscosity gradient in the liquid within the disruptate, and separating the refractile material from the cellular debris by high-speed centrifugation. The IL-2 is then solubilized with a solubilizing agent such as SDS, chromatographed to remove high molecular weight contaminants, oxidized, and purified by a combination of HPLC, ultrafiltration and gel filtration.

An abstract titled "Purification and Renaturation of Recombinant Interleukin-2" presented at the 6th International Symposium on HPLC of Proteins, Peptides and Polynucleotides at Baden-Baden, West Germany in October 1986 describes a process in which recombinant IL-2 is solubilized from inclusion bodies with 6 M guanidine hydrochloride/10 mM dithiothreitol (DTT) and purified in a reduced, denatured form by FPLC gel permeation. The solution from the FPLC gel permeation is diluted to effect renaturation and autooxidation. In this regard U.S. Patents Nos. 4,511,502; 4,511,503; 4,512,922 and 4,518,526; and EP publication 114,506 describe a similar procedure for purifying heterologous proteins in general from refractile bodies. In such processes, the oxidation and renaturation of the IL-2 are carried out in a single step. However, because of disparate solubility characteristics between the reduced and oxidized forms of IL-2, it is difficult to achieve high yields of renatured oxidized IL-2 in such a process.

EP publication 145,390 describes a process for recovering rIL-2 from E. coli in which the cells are suspended in 7 M guanidine hydrochloride, solids are removed by centrifugation, the rIL-2-containing supernatant is dialyzed to remove the guanidine hydrochloride and the dialyzate is purified by anion exchange chromatography, gel filtration and RP-HPLC.

The present invention is directed to an improved recombinant IL-2 purification process in which the oxidation and renaturation are carried out in distinct steps.

### Disclosure of the Invention

The present invention relates to a high yield process in which IL-2 is separated from a cellular disruptate in the form of a refractile body, dissolved with a chaotropic agent and oxidized and renatured in separate steps followed by purification to a clinically acceptable level.
One aspect of the invention is a process for recovering recombinant IL-2 from a transformed microorganism containing the IL-2, comprising:
(a) isolating water insoluble IL-2 containing material;
(b) dissolving and denaturing the IL-2 in said water insoluble IL-2 material in the presence of a reducing agent and a strongly denaturing concentration of a chaotropic agent;
(c) reducing the concentration of the chaotropic agent so that the reduced and denatured IL-2 precipitates;
(d) solubilizing the IL-2 precipitate in a sufficient concentration of chaotropic agent to keep the reduced and denatured IL-2 in solution;
(e) oxidizing the solubilized IL-2 while maintaining the concentration of chaotropic agent at a strongly denaturing concentration to form the natural disulfide bridge in IL-2;
(f) reducing the concentration of chaotropic agent to a level at which the oxidized IL-2 is permitted to renaturate and refold into the configuration of native IL-2 and at which insoluble extraneous host proteins precipitate; and
(g) separating and removing said insoluble proteins from the soluble renatured, oxidized IL-2.
In another aspect, the invention relates to a process for recovering purified soluble recombinant IL-2 from a transformed microorganism containing the IL-2 comprising:
(a) disrupting the cell membrane and cell wall of the microorganism;
(b) separating water insoluble material from the disruptate;
(c) mixing the insoluble material of step (b) at a pH of about 7 to about 9 with an aqueous solution of a reducing agent and a strongly denaturing concentration of a chaotropic agent whereby the IL-2 in the insoluble material is dissolved;
(d) precipitating the IL-2 out of the IL-2-containing solution and recovering the precipitate;
(e) solubilizing the IL-2 precipitate;
(f) oxidizing the IL-2 in the solution whereby the natural disulfide bridge of IL-2 is formed;
(g) after the oxidation of step (f) is complete, reducing the concentration of chaotropic agent in the solution to a level at which the oxidized IL-2 is renaturated and a precipitate forms;
(h) separating the precipitate of step (g) from the solution to provide a supernatant;
(i) purifying the oxidized, renatured IL-2 in the supernatant by reverse-phase high performance liquid chromatography followed by dissolution of the pool in a solution of chaotropic agent and removal of the chaotropic agent from the solution, or by hydrophobic interaction chromatography combined with ion exchange chromatography, or by ion exchange chromatography; and
(j) recovering a purified oxidized, soluble heterologous human IL-2 composition having an IL-2 content of at least about 95% as determined by reducing sodium dodecyl sulfate polyacrylamide gel electrophoresis analysis, a solubility of at least 5 mg IL-2 per ml, a specific activity of at least 1 x 10⁷ units/mg as determined by HT-2 cell proliferation assay, and an endotoxin content of less than about 0.1 nanograms per mg of IL-2. Preferably the composition is also substantially free of pyrogens as determined by the U.S.P. rabbit pyrogen test at a dosage of 1.0 x 10³ units per kg, more preferably 3.3 x 10⁵ units per kg.

### Brief Description of the Drawings

Figure 1 is a flow diagram of the preferred embodiment of the invention process.

Figure 2 is a graph of the results of the immunoassay described in Example 1, infra. The closed circles represent the SDS-process IL-2 and the closed squares represent the guanidine-process IL-2, both of which are described in the example.

### Modes for Carrying Out the Invention

### A. Definitions

As used herein, the term "IL-2" refers to recombinant interleukin-2 or interleukin-2-like polypeptides produced by a transformed microorganism and whose amino acid sequence is the same as or similar or substantially homologous to the unglycosylated and/or glycosylated native interleukin-2. Examples of such recombinant IL-2s are those described in European published patent applications 91,539, 88,195, and 109,748, as well as those described in U.S. Patent No. 4,518,584,

EP publication 200,280, and bovine IL-2 as described by Cerretti et al, Proc Natl Acad Sci USA (1986) 83:3223-3227.

The recombinant IL-2s particularly preferred herein are those biologically active muteins (analogs) of human IL-2 in which amino acid residues not essential to biological activity have been deliberately deleted in some instances or replaced with a conservative amino acid, as indicated below. More specifically, preferred recombinant IL-2s include those wherein the cysteine residue at position 125 is replaced with another amino acid, preferably neutral or conservative, to eliminate sites for intermolecular crosslinking or incorrect intramolecular disulfide and, optionally, the N-terminal alanine residue of the native counterpart is eliminated. As used herein, such neutral or conservative amino acids are glycine, serine, valine, alanine, leucine, isoleucine, tyrosine and methionine. More particularly, preferred recombinant IL-2 muteins in the formulations of this invention are those wherein (1) the cysteine residue at amino acid position 125 of the native counterpart is replaced by a serine residue (designated IL-2ₛₑᵣ₁₂₅) or alanine residue (designated IL-2ₐ₁ₐ₁₂₅); or (2) the initial alanine residue is eliminated and the cysteine at position 125 is replaced by serine (designated des-alanyl-IL-2ₛₑᵣ₁₂₅).

Other IL-2s particularly preferred herein are those biologically active muteins described in European Patent Publication 200,280 wherein oxidation-susceptible methionine residues are replaced with a neutral or conservative amino acid; a preferred mutein includes replacing the methionine at position 104 with a conservative amino acid such as alanine.

EP 200,280 also describes amino-terminal deletions of IL-2 wherein one or more of the first six amino acids are deleted. Preferred oxidation-resistant muteins include ala₁₀₄ser₁₂₅IL-2, ala₁₀₄IL-2, ala₁₀₄ala₁₂₅IL-2, val₁₀₄ser₁₂₅IL-2, val₁₀₄IL-2, val₁₀₄ala₁₂₅IL-2, des-ala₁ala₁₀₄ser₁₂₅IL-2, des-ala₁ala₁₀₄IL-2, des-ala₁ala₁₀₄ala₁₂₅IL-2, des-ala₁val₁₀₄ser₁₂₅IL-2, des-ala₁val₁₀₄IL-2, des-ala₁val₁₀₄ala₁₂₅IL-2, des-ala₁des-pro₂ala₁₀₄ser₁₂₅IL-2, des-ala₁-des-pro₂ala₁₀₄IL-2, des-ala₁des-pro₂ala₁₀₄ala₁₂₅IL-2, des-ala₁des-pro₂val₁₀₄ser₁₂₅IL-2, des-ala₁des-pro₂-val₁₀₄IL-2, des-ala₁des-pro₂val₁₀₄ala₁₀₄IL-2, des-ala₁des-pro₂des-thr₃ala₁₀₄ser₁₂₅IL-2, des-ala₁des-pro₂des-thr₃ala₁₀₄IL-2, des-ala₁des-pro₂-des-thr₃ala₁₀₄ala₁₂₅IL-2, des-ala₁des-pro₂des-thr₃-val₁₀₄ser₁₂₅IL-2, des-ala₁des-pro₂des-thr₃val₁₀₄IL-2, des-ala₁des-pro₂des-thr₃val₁₀₄ala₁₂₅IL-2, des-ala₁des-pro₂des-thr₃des-ser₄ala₁₀₄ser₁₂₅IL-2, des-ala₁des-pro₂des-thr₃des-ser₄ala₁₀₄IL-2, des-ala₁des-pro₂des-thr₃des-ser₄ala₁₀₄ala₁₂₅IL-2, des-ala₁des-pro₂des-thr₃des-ser₄val₁₀₄ser₁₄₅IL-2, des-ala₁des-pro₂des-thr₃des-ser₄val₁₀₄ser₁₂₅IL-2, des-ala₁des-pro₂des-thr₃des-ser₄val₁₀₄ala₁₂₅IL-2, des-ala₁des-pro₂des-thr₃des-ser₄des-ser₅ala₁₀₄ser₁₂₅IL-2, des-ala₁des-pro₂des-thr₃des-ser₄des-ser₅ala₁₀₄IL-2, des-ala₁des-pro₂des-thr₃des-ser₄des-ser₅ala₁₀₄ala₁₂₅-IL-2, des-ala₁des-pro₂des-thr₃des-ser₄des-ser₅val₁₀₄-ser₁₂₅IL-2, des-ala₁des-pro₂des-thr₃des-ser₄des-ser₅-val₁₀₄IL-2, des-ala₁des-pro₂des-thr₃des-ser₄des-ser₅-val₁₀₄-ala₁₂₅IL-2, des-ala₁des-pro₂des-thr₃des-ser₄-des-ser₅des-ser₆ala₁₀₄ala₁₂₅IL-2, des-ala₁des-pro₂-des-thr₃des-ser₄des-ser₅des-ser₆ala₁₀₄IL-2, des-ala₁des-pro₂des-thr₃des-ser₄des-ser₅des-ser₆ala₁₀₄ser₁₂₅IL-2, des-ala₁des-pro₂des-thr₃des-ser₄des-ser₅-des-ser₆val₁₀₄ser₁₂₅IL-2, des-ala₁des-pro₂des-thr₃-des-ser₄des-ser₅des-ser₆val₁₀₄IL-2, or des-ala₁des-pro₂des-thr₃des-ser₄des-ser₅des-ser₆val₁₀₄-ala₁₂₅IL-2.

Other amino-terminal deletions of IL-2 are disclosed in Chemical Abstracts (1987) 106:(21):170236f, an abstract of Japanese Patent Publication No. 61/225199, published 6 October 1986, wherein any one of the first 15 amino acids of IL-2 are deleted. PCT 87/04714, published 13 August 1987 describes deletions or replacements of one or more of the amino acid residues in positions 2 to 11 and/or 128 to 133 from the amino-terminal alanine of IL-2.

The precise chemical structure of the IL-2 protein will depend on a number of factors. As ionizable amino and carboxyl groups are present in the molecule, a particular recombinant IL-2 protein may be obtained as an acidic or basic salt, or in neutral form. All such preparations which retain their activity when placed in suitable environmental conditions are included in the definition of IL-2 proteins herein. Further, the primary amino acid sequence of the protein may be augmented by derivatization using sugar moieties (glycosylation) or by other supplementary molecules such as lipids, phosphate, acetyl groups and the like, more commonly by conjugation with saccharides. Certain aspects of such augmentation are accomplished through post-translational processing systems of the producing host; other such modifications may be introduced in vitro. In any event, such modifications are included in the definition of IL-2 protein herein so long as the activity of the protein, as defined above, is not destroyed. It is expected, of course, that such modifications may quantitatively or qualitatively affect biological activity, either by enhancing or diminishing the activity of the protein in the various assays.

As used herein the term "transformed" in describing host microorganism cell cultures denotes a microorganism that has been genetically engineered to produce an IL-2 polypeptide that is capable of possessing the activity of native IL-2. Bacteria are preferred microorganisms for producing the IL-2 protein. E. coli is particularly preferred.

The term "chaotropic agent" refers to a compound or compounds which, in aqueous solution and in a suitable concentration, are capable of denaturing recombinant IL-2. Correlatively the term "strongly denaturing concentration" refers to a solution of a chaotropic agent which will effectively "unfold" or denature recombinant IL-2. Guanidine salts (e.g. the hydrochloride) and alkali metal thiocyanate (e.g. sodium thiocyanate) at concentrations in the range of about 4 to 9 M, preferably 6-9 M, are examples of chaotropic agent solutions that will dissolve and denature recombinant IL-2.

### Cell Growth

The IL-2-producing transformed microorganisms are grown in a suitable growth medium, typically to an optical density (OD) of at least about 30 at 680 nm, and preferably between about 20 and 40 at 680 nm. The composition of the growth medium will depend upon the particular microorganism involved. The medium is an aqueous medium containing compounds that fulfill the nutritional requirements of the microorganism. Growth media will typically contain assimilable sources of carbon and nitrogen, energy sources, magnesium, potassium and sodium ions, and optionally amino acids and purine and pyrimidine bases. (See Review of Medical Biology, Lange Medical Publications, 14th Ed pp. 80-85 (1980).) In expression vectors involving the trp promoter, the tryptophan concentration in the medium is carefully controlled to become limiting at the time protein expression is desired. Growth media for E. coli are well known in the art.

After the cells are harvested from the culture, they may be concentrated, if necessary, to about 20 to 150 mg/ml, preferably 80 to 100 mg/ml (OD 40 to 300, preferably 160 to 200 at 680 nm) by cross-flow filtration, centrifugation, or other conventional methods.

### Cell Disruption

Following concentration of the harvested culture, the cell membranes and cell walls of the microorganisms are disrupted. Preferably a compound which is non-toxic to humans, such as 1-octanol, in an amount of about 1% by weight of total components, is added to the disrupted cells to ensure that no viable recombinant organisms remain. Conventional cell disruption techniques such as homogenization, sonication, or pressure cycling may be used in this step of the process. The end point of the disruption step can be determined by monitoring the optical density with the absorbance at 260 nm of the suspension typically increasing with cell lysis and by microscopic observation. In any event, the disruption should break substantially all of the cells so that substantially no intact cells are carried through to subsequent steps.

### Treatment of Disruptate to Isolate Insoluble IL-2 Bodies

After the cells have been disrupted, deionized water is preferably added to the disruptate and greater than 99% by weight of the salts are removed therefrom. The salts are water-soluble materials composed of oppositely charged small molecular weight ions. The removal of these salts to reduce the ionic strength of the disruptate may be accomplished by diafiltration using deionized water to flush out the ions or by centrifuging. to pellet the cellular debris and refractile bodies followed by resuspension in deionized water. If diafiltration is employed, preferably deionized water is continuously added such that the rate of addition of water equals the filtration rate.

After the salts are essentially removed, optionally a compound such as 1-octanol may be added to the desalted disruptate, if not added earlier, to ensure that no viable recombinant organisms remain before containment is broken. The desalted disruptate is again disrupted as described above for the initial disruption.

After redisruption, density or viscosity is increased and/or a gradient is created during centrifugation in the liquid within the disruptate by adding a material to the disruptate. There are several means to accomplish this purpose, all relying on the sedimentation characteristics of the particles by varying the density and/or viscosity of the liquid phase. One means to accomplish this goal is to add a material which increases the density of the liquid to a ρ of about 1.1 to 1.3 g/ml, preferably 1.13 to 1.17 g/ml.

Materials which may be used to accomplish this density increase include a sugar or mixture of sugars, such as, e.g., sucrose, dextrose, fructose, maltose, maltotriose, and other mono-, dior polysaccharides. Most preferably the sugar is sucrose. Alternatively, a two-phase system of materials such as, e.g., a glycerol/sucrose mixture may be used wherein the disrupted particles partition to the interface between the heavy and light phases and can be eluted by a liquid/liquid separation.

In addition, the viscosity of the liquid phase may be increased to from 5 to 10 cps by any suitable means such as by adding a viscous compound such as, e.g., sucrose or glycerol thereto. Also, a gradient is created if, e.g., the particles are in a 60% aqueous glycerol suspension while the centrifuge bowl contains 80% aqueous glycerol.

The IL-2-containing refractile bodies are separated from the cellular debris by high-speed centrifugation. By "high-speed centrifugation" is meant spinning the suspension in a centrifuge at about 8,000 to 40,000 times gravity (g), preferably about 10,000-20,000 x g, for a suitable time period depending on the volume, generally about 10 minutes to seventy-two hours.

The particle pellet or paste resulting from the centrifugation contains approximately 15-70% by weight IL-2 as determined by SDS-polyacrylamide gel electrophoresis and by Lowry assay (Lowry et al, J Biol Chem (1951) 193:265-275).

The IL-2 in the particle paste or pellet is then dissolved and denatured by mixing the paste/pellet with a solution of a strongly denaturing concentration of the chaotropic agent and a reducing agent. The chaotropic agent and reducing agent are in an aqueous buffer at pH 7 to 9, preferably phosphate buffered saline or Tris buffer. pH adjustment may be accomplished by the addition of base such as NaOH. The w/v ratio of pellet to solution will normally be in the range of 0.01:1 to 0.25:I preferably 0.05:1 to 0.12:1. Reducing agents that can be employed during the dissolving/denaturing step include: B-mercaptoethanol, glutathione, cysteine and dithiothreitol (DTT). DTT is the preferred reducing agent. The concentration of the reducing agent in the medium will usually range between about 10 to 100 mM with approximately 50 mM being the preferred concentration. Chelating agents such as ethylene diaminetetraacetic acid (EDTA) in concentrations of 1 to 50 mM, preferably about 25 mM, and buffers such as Tris·HCl at concentrations of 25 to 250 mM, preferably 50 mM, may be included in the solution. Elevated temperatures in the range of 35°C to 50°C, preferably about 40°C, and a nitrogen blanket may be used in this step. The dissolution/denaturation will typically be complete after about 5 to 15 min of mixing After this time, the mixture may be centrifuged, preferably at 2000 x g to 4000 x g for about 10 to 30 min to remove any undissolved materials.

The reducing agent, along with other contaminating material, is first removed using gel filtration, diafiltration, or precipitation. Gels that are capable of removing the reducing agent from the protein solution are commercially available. For instance, when DTT is used as a reducing agent Sephadex" G-10, G-25, and G-50 gels may be used. The gel filtration will be run in a solution of the chaotropic agent that maintains the protein in solution. When guanidine hydrochloride is used, concentrations above about 6 M are required to keep the IL-2 in solution and avoid precipitate formation. After removing the reducing agent the protein solution is diluted, if necessary, with the solution of chaotropic agent to a protein concentration of about 0.1 to 2 mg/ml, preferably about 0.25 to 1.0 mg/ml.

A preferred method of removing the reducing agent along with contaminating material employs precipitation techniques. The reduced IL-2 is diluted to approximately 2-30 mg of IL-2 per ml, preferably 5-10 mg of IL-2 per ml with 7.0 M guanidine. This is then diluted to approximately 1-5 M guanidine, preferably 3.5 M guanidine, and allowed to stand until the precipitation is complete, generally two hours. Once the precipitate begins to settle, the IL-2 can be pelleted by centrifugation. Residual DTT may be removed by washing the pellet with a buffer, preferably using a 2-4 M guanidine-containing buffer or a 1-2% polysorbate 80-containing buffer.
The denatured IL-2 is next subjected to a controlled oxidation.

Preferred selective oxidation procedures are described in commonly owned U.S. Patents Nos. 4,572,798 (using an oxidation promoter containing a Cu⁺² cation such as from CuCl₂, Cu(NO₃)₂, etc) and 4,530,787 (using o-iodosobenzoic acid).

The Cu⁺² oxidation comprises reacting the aqueous solution of denatured IL-2 at a pH between about 5.5 and 9, preferably 6 to 8, and most preferably about 7.5, in the presence of air width at least an effective amount of an oxidation promoter containing a Cu⁺² cation. Controlled oxidation causes the formation of disulfide bridging in the IL-2 which conforms to the bridging in native IL-2 with no or minimal overoxidation and formation of nonconforming bridging or oligomers. Such oxidation enables the production of high yields of the recombinant IL-2 with the proper disulfide bridging.

The amount of oxidant or oxidation promoter employed is at least an effective amount for oxidation, i.e., an amount which at minimum will be necessary to conduct the oxidation reaction effectively within a convenient period of time. An effective amount is the amount approximately equivalent to the concentration of free sulfhydryl groups in the IL-2 which are destined to be involved in forming the desired disulfide bonds. Preferably, the amount of CuCl₂ will range from about 5 to 275 micromolar. In the case of o-iodosobenzoic acid the mole ratio of oxidant to IL-2 will preferably be in the range of about 0.05:1 to about 5:1, most preferably about 0.8:1 to about 1:2. The concentration of IL-2 in the reaction mixture is kept low, i.e., generally less than about 5 mg/ml, preferably about 0.05 to about 2 mg/ml, and more preferably about 0.1 to about 1 mg/ml, to reduce the likelihood of oligomer formation. The pH is maintained between 5.5 and 9, preferably between 7 and 8 in the o-iodosobenzoic acid oxidation.

The reduced IL-2 must remain in solution for effective oxidation to occur. Therefore, the reaction mixture must contain a sufficient concentration of chaotropic agent to keep the reduced IL-2 in solution. As indicated above, when guanidine hydrochloride is used, its concentration must be above 6 M. At such concentrations, a substantial amount of the IL-2 will be in a denatured form. For this reason, it is difficult in the case of IL-2 to carry out the oxidation and renaturation simultaneously and obtain high yields of renatured IL-2.

The temperature used in the oxidation will normally be between about 20°C and 40°C, conveniently room temperature. For Cu⁺² oxidation, increasing the reaction temperature increases the rate of reaction. The oxidation reaction may be effectively terminated by, e.g., lowering the pH to a level at which the reaction ceases, freezing the solution, or adding chelators such as EDTA to the reaction mixture. Oxidation time will normally be in the range of 4 hr to about one day.

When the oxidation is complete, the concentration of the chaotropic agent (guanidine hydrochloride) is reduced using dilution, dialysis, or diafiltration, to a level which permits the oxidized IL-2 to renaturate and refold into the configuration of native IL-2. Phosphate buffer or Citrate buffer, 10 to 100 mM, preferably about 10 mM; NaCl, 10-150 mM, preferably 40 mM; and sucrose, 1-5%, preferably 2.5%, are preferred diluents. Preferably, the IL-2 is concentrated wing an ultrafiltration membrane to avoid handling large volumes of solution. The concentration of guanidine hydrochloride agent is normally diluted or diafiltered to below about 2 M, preferably below about 0.5 M. The dilution will typically be carried out at about 4°C to 25°C. At such temperatures and reduced guanidine hydrochloride concentration a precipitate of extraneous host protein forms. This precipitate is removed by filtration or centrifuging to provide a supernatant containing the oxidized, renaturated IL-2.

The renatured, oxidized IL-2 is then purified to remove endotoxins to a level that meets clinical specifications (i.e., less than about 0.1 ng endotoxin per ml of IL-2). The IL-2 is also preferably purified to remove pyrogen so as to be substantially free of pyrogens as measured by the U.S.P. rabbit pyrogen test at a dosage of 1.0 x 10³ units/kg, preferably 3.3 x 10⁵ units/kg). The purification may be achieved by ion exchange chromatography, or a combination of hydrophobic interaction and ion exchange chromatography, or by RP-HPLC.

The solution is then flowed over an ion exchange column, such as a DEAE agarose column (e.g., Pharmacia Fast-Flow Sepharose (registered Trade Mark) DEAE). IL-2 is recovered from the column with 10 mM citrate, pH 6.5. The eluted IL-2 fractions may subsequently be loaded onto another ion exchange column such as a carboxymethyl agarose column (e.g., Pharmacia Fast-Flow Sepharose CM) that binds IL-2 at a pH of 6 to 7.5. The bound IL-2 may be eluted with an increasing salt gradient. The desired IL-2 elutes at approximately 150 mM salt with the lower isoelectric point forms of the protein eluting at lower salt concentrations.

In the hydrophobic interaction/ion exchange chromatography technique, (NH₄)₂SO₄ is added to the IL-2 solution to a concentration of at least about 1.0 M, preferably about 1.25 M. The solution is then loaded onto a hydrophobic interaction column, such as a phenyl agarose column (e.g., Pharmacia Phenyl Fast-Flow Sepharose column). Bound IL-2 is recovered from the column with a decreasing (NH₄)₂SO₄ gradient, with the IL-2 being collected in the fractions eluting at about 0.95 to 0.75 M (NH₄)₂SO₄. Species of IL-2 and other impurities (bacterial host proteins) having lower isoelectric points than native IL-2 are then removed by cation exchange chromatography using an exchanger that binds IL-2 at a pH of 6 to 7.5. A carboxymethyl agarose column (e.g., Pharmacia Fast-Flow Sepharose CM) is a preferred preparative cation exchanger. The solution is contacted with the exchanger at the indicated pH range and the IL-2 is eluted from the exchanger using an ionic gradient. The desired IL-2 elutes at approximately 0.15 M salt with the lower isoelectric point forms of the protein eluting at lower salt concentrations.

The HPLC purification of the renatured IL-2 may be carried out in essentially the same manner as described in U.S. 4,569,790 followed by redissolution in a chaotropic agent and dialysis. Briefly, the solution of IL-2 is chromatographed, precipitated, and the resulting precipitate is taken up in the chaotropic agent solution. The chaotropic agent is then removed by dialysis or diafiltration. The IL-2 may be further purified by cation exchange chromatography.

The purity of the renatured, oxidized IL-2 after the chromatography steps is at least about 95% and usually at least about 98%, as determined by reducing sodium dodecyl sulfate polyacrylamide gel electro phoresis (SDS-PAGE) analysis. This pure IL-2 has a solubility in PBS of at least about 5 mg/ml, a specific activity of at least about 1 x 10⁷ units/mg, usually 5 x 10⁶ to 2 x 10⁷ units/mg as determined by the HT-2 cell proliferation assay, and endotoxin content of less than about 0.1 ng/mg of IL-2. Also, preferably the IL-2 is substantially free of pyrogens as determined by the U.S.P. rabbit pyrogen test at a dosage of 1.0 x 10³ units/kg, more preferably 3.3 x 10⁵ units/kg.

### Formulation

The purified IL-2 is rendered aqueous, its concentration is adjusted, if necessary, to 0.01 to 2 mg/ml, and a water-soluble carrier is added to the desired level. The carrier will typically be added such that it is present in the solution at about 1% to 10% by weight, preferably about 5% by weight. The exact amount of carrier added is not critical. Conventional solid bulking agents that are used in pharmaceutical tablet formulation may be used as the carrier. These materials are water soluble, do not react with the IL-2, and are themselves stable. They are also preferably non-sensitive to water (i.e., nonhygroscopic). Specific examples of carriers that may be added include dextrose, lactose, mannitol, sucrose, and other reduced sugars such as sorbitol, starches and starch hydrolysates derived from wheat, corn, rice, and potato, microcrystalline ce-lluloses, and albumin such as human serum albumin. Mannitol, sucrose, and dextrose are preferred.

The carrier adds bulk to the formulation such that when unit dosage amounts of the solution are lyophilized in containers, such as sterile vials, the freeze-dried residue will be clearly discernible to the naked eye. In this regard the preferred carrier, mannitol, yields an aesthetically acceptable (white, crystalline) residue that is not sensitive to water. The nonsensitivity of mannitol to water may enhance the stability of the formulation.

EP publication 215,658, published 25 March 1987, entitled "An Improved Formulation for Lipophilic Proteins" (Hanisch et al) outlines an improved process for recovering and purifying lipophilic recombinant proteins such as IL-2 from microorganisms to yield a protein preparation which may be formulated into a stable pharmaceutical composition. Such a composition carrying a therapeutically effective amount of the biologically active recombinant lipophilic protein dissolved in a non-toxic, insert, therapeutically compatible aqueous-based carrier medium at a pH of 6.8 to 7.8 also contains a stabilizer for the protein, such as human serum albumin, normal serum albumin and human plasma protein fraction. The formulation aspects of said EP publication 215,658 are
an alternative formulation route for the purified IL-2. EP publication 215,658 outlines a low pH formulation process. U.S. Patent No. 4,462,940 to Hanisch et al, outlines a high pH formulation process, and the formulation aspects thereof are also herein incorporated by reference.

After adding the carrier, the unit dosage amounts (i.e., for IL-2 volumes that will provide 0.01 to 2 mg, preferably 0.2 to 1.0 mg, IL-2 per dose) of the solution are dispensed into containers, the containers are capped with a slotted stopper, and the contents are lyophilized using conventional freeze-drying conditions and apparatus.

The lyophilized, sterile product consists of a mixture of (1) IL-2, (2) carrier (dextrose, sucrose, or mannitol), (3) optionally other excipients such as human serum albumin, Tween® 80, and the like and (4) a small amount of buffer that will provide a physiological pH when the mixture is reconstituted. The product may also contain a minor amount of a preservative to enhance chemical stability. The recombinant IL-2 will typically constitute about 0.015% to 10% by dry weight of the mixture, more preferably about 2% to 5% of the mixture.

The lyophilized mixture may be reconstituted by injecting a conventional parenteral aqueous injection such as distilled water for injection, Ringer's solution injection, Hank's solution injection, dextrose injection, dextrose and salt injection, physiological saline injection, or the like, into the vial. The injection should be added against the side of the vial to avoid excess foaming. The amount of injection added to the vial will typically be in the range of 1 to 5 ml, preferably 1 to 2 ml.

In an alternative formulation, described in PCT WO87/00056, published 15 January 1987, entitled "Solubilization of Recombinant Proteins for Pharmaceutical Compositions Using Homopolymer Conjugation" to M. Knauf et al,

the IL-2 is reacted with an activated polymer selected from polyethylene glycol, homopolymers and polyoxyethylated polyols such as polyoxyethylated glycerol. The polymer preferably has a molecular weight of from 300 to 100,000 daltons, more preferably 350 to 40,000 daltons. The polymer is activated by conjugation with a coupling agent having terminal groups reactive with both the free amine or thiol groups of the protein and the hydroxyl group of the polymer. Examples of such coupling agents include hydroxynitrobenzene sulfonic ester, cyanuric acid chloride, and N-hydroxysuccinimide. The IL-2 is then formulated directly with the water-soluble carrier and buffer as described above, the formulation is lyophilized, and the lyophilized mixture may be reconstituted as described above.

The reconstituted formulation prepared as described above is suitable for parenteral and oral administration to humans or other mammals in therapeutically effective amounts (i.e., amounts which eliminate or reduce the patient's pathological condition) to provide therapy thereto. IL-2 therapy is appropriate for a variety of immunomodulatory indications such as T cell mutagenesis, induction of cytotoxic T cells, augmentation of natural killer cell activity, induction of IFN-γ, restoration and enhancement of cellular immunity (e.g., treatment of immune deficient conditions), and augmentation of cell-mediated anti-tumor activity.

The formulations of this invention are useful for parenteral administration, for example, intravenous, subcutaneous, intramuscular, intraorbital, ophthalmic, intracapsular, intraspinal, intrasternal, topical, intranasal aerosol, scarification, and also, for oral administration. The preferred routes of administration are by intramuscular, subcutaneous and intravenous injection, and by topical administration. The use of nonionic detergents are especially preferred for topically administered formulations because of their ability to penetrate the skin surface.

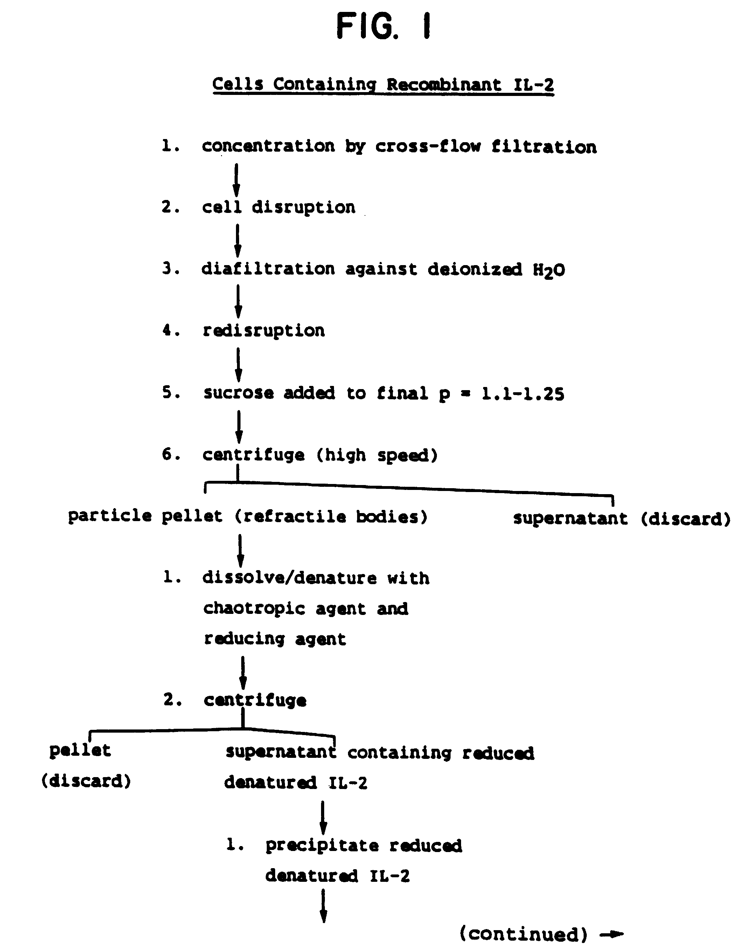

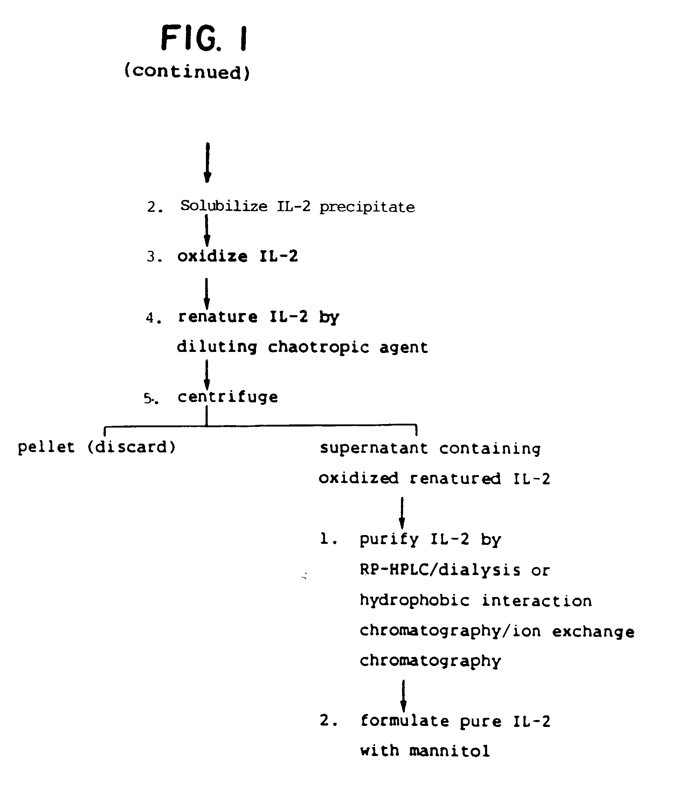

The following examples further illustrate the process and composition of the invention. These examples are not intended to limit the invention in any manner. In these examples all temperatures are in degrees Celsius unless otherwise indicated. Figure 1 indicates the preferred process of this invention, represented by the examples.

### Example 1

This example illustrates a preferred process for recovering, purifying and formulating recombinant IL-2.

Des-alanyl-IL-2ₛₑᵣ₁₂₅ was recovered from E. coli. The strain of des-alanyl-IL-2ₛₑᵣ₁₂₅-producing E. coli (K12/MM294-1) carrying plasmid pLW45 used in this example was deposited at the American Type Culture Collection of 4 March 1984 under accession number 39,626. Said analog and a method of preparation are disclosed in U.S. Patent No. 4,518,584.

The E. coli thus transformed with plasmid pLW45 were grown in a 1000-liter fermenter at 37°C. The dissolved oxygen was maintained at about 40% by, as necessary, (1) increasing agitation; (2) adding air; and (3) adding oxygen.

Once the fermenter was filled with water to the operating volume, the following trace elements were added:

| | |
|---|---|
| ZnSO₄ · 7H₂O | 30 µM |
| MnSO₄ · 4H₂O | 30 µM |
| CuSO₄ · 5H₂O | 3 µM |
| Na₃ citrate · 2H₂O | 1.5 mM |
| KH₂PO₄ | 21 mM |
| (NH₄)₂SO₄ | 72 mM. |

The fermenter feed and addition vessels were then sterilized according to standard operating procedures. Then the following sterile additions were made:

| | |
|---|---|
| MgSO₄ · 7H₂O | 3 mM |
| FeSO₄ · 7H₂O | 72 µM |
| L-tryptophan | 70 mg/L |
| thiamine · HC | 20 mg/L |
| glucose | 50 g/L |
| tetracycline | 5 mg/L. |

The fermenter was cooled and inoculated with frozen or seed E. coli culture at 2 mg/L dry weight cells. Throughout fermentation, the pH is maintained at 6.8 using KOH. Optical density measurements and residual glucose measurements on samples were taken at 14-16 hours and approximately one hour intervals thereafter.

Induction of des-alanyl-IL-2ₛₑᵣ₁₂₅ production by depletion of L-tryptophan from the culture medium occurred at about OD₆₈₀=10 followed by the addition of casamino acids to a final concentration of 2% at OD₆₈₀=15. Cultures were harvested about 3-5 hours later.

The refractile bodies containing the desalanyl-IL-2-ser₁₂₅ were then isolated. The harvested material was concentrated about 5-10 fold by circulating the harvest material under pressure through UF cross-flow filtration cartridges with a 100 K molecular weight cutoff. The cells were disrupted by 3 passes through a disrupter at about 6500 psi (6.895 x 10³Nm⁻²) (195 atm (1.98 x 10⁷Pa)). EDTA was then added to a final concentration of 5 mM. The suspension was diafiltered against 5 volumes of deionized water. Octanol was added to 1% (v/w) to kill any residual live bacteria in the diafiltered product. 2 mM EDTA was added and after several hours, the diafiltered disruptate was redisrupted by passing it through a disrupter.

Sucrose was added to the redisruptate to create a final density between 1.1 and 1.25 g/ml. The mixture was centrifuged at 8,000 to 20,000 x g at 1-2 1pm, and the particle pellet or paste was collected. A temperature of at least 20°C was maintained prior to and during centrifugation.

The particle paste was then mixed with 17 ml per gram of paste of an aqueous solution of saturated guanidine hydrochloride, DTT, 50 mM, Tris, 50 mM, and 25 mM EDTA, the pH was adjusted to 8.0 with NaOH and heated to 40°C for about 10 min. Undissolved materials were removed from the mixture by centrifugation at 3000 x g for 15 min.

The next step in the purification was to remove the DTT and EDTA from the IL-2 solution (supernatant) by gel filtration using a Sephadex" G-25 column. The column was run in 7 M guanidine hydrochloride buffer at pH 7.5. Using a process chromatogram, the IL-2 peak was collected and the peak was diluted with guanidine hydrochloride buffer to a protein concentration of 0.5 mg/ml.

Oxidation of the IL-2 was initiated by adding CuCl₂ in a molar ratio of 3:1 (CuCl₂ to IL-2). The oxidation was carried out at about 25°C in 7 M guanidine hydrochloride, 10 mM phosphate. The pH was controlled at 7.5 ± 0.2 during oxidation and 4 mM EDTA was added when the oxidation was completed. Since oxidized IL-2 is more hydrophilic than reduced IL-2, the progress of the oxidation reaction was monitored by RP-HPLC.

The resulting solution of oxidized IL-2 was then diluted with 10 mM phosphate buffer to reduce the guanidine hydrochloride concentration to 2 M. The IL-2 concentration was then increased to 2.5 mg/ml using a hollow fiber membrane ultrafiltration unit with a 10,000 dalton cutoff. The solution was then further diluted with 10 mM phosphate buffer to 0.2 M guanidine hydrochloride and allowed to sit overnight at 4°C to obtain a precipitate.

The precipitate consisting of extraneous E. coli proteins and some IL-2 was then removed by filtering with a cellulose acetate filter to obtain approximately 85% recovery of refolded IL-2. (NH₄)₂SO₄ was then added to the supernatant to a concentration of 1.25 M. This solution was loaded onto a Pharmacia Phenyl Fast-Flow Sepharose hydrophobic interaction column. IL-2 was recovered from the column with a decreasing (NH₄)₂SO₄ gradient with the IL-2 collected in the fractions at about 0.95 to 0.75 M (NH₄)₂SO₄. The pooled fractions were diafiltered and then loaded on a Pharmacia carboxymethyl (CM) Fast-Flow Sepharose ion exchange column at pH 7 equilibrated with 10 mM phosphate buffer. IL-2 fractions were recovered at about 0.15 M NaCl.

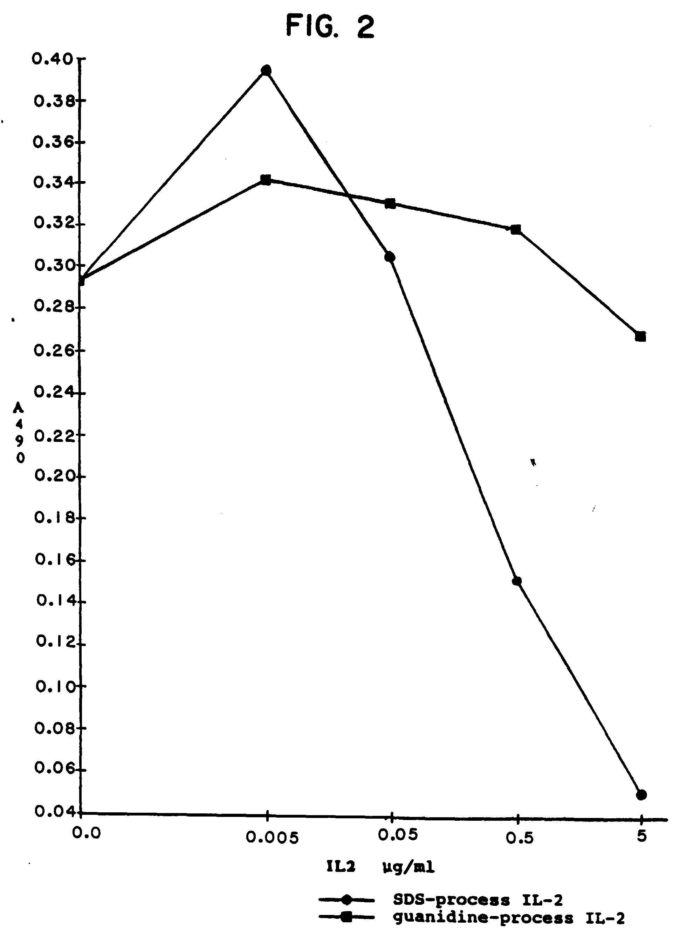

The resulting IL-2 was 98% pure by SDS-PAGE analysis and homogeneous by HPLC analysis. Its specific activity was 8 x 10⁶ units/mg as measured by the HT-2 cell proliferation assay. Enzyme-linked immunosorbent assays (ELISA) were carried out to determine whether this renatured recombinant IL-2 (guanidine-process IL-2) binds to polyclonal antibodies that bind to the recombinant IL-2 made by the process described in U.S. Patent No. 4,569,790,
in which the IL-2 is solubilized with SDS (SDS-process IL-2). Serum from a patient treated with SDS-process IL-2 was diluted 1:1000 in assay buffer (PBS with 0.5% BSA and 0.05% Tween 20) and mixed with SDS or guanidine-process IL-2 in final concentrations of 0 to 5 µg/ml. After a two hour room temperature incubation, the mixtures were applied in 100 µL volumes to 96 well microelisa plates (Immulon (registered Trade Mark) I, Dynatech) previously coated with SDS-process IL-2 (5 µg/ml in 0.05 M Na₂CO₃, pH 9.6, 100 µl/well). The mixtures were allowed to sit in the IL-2-coated wells for thirty minutes, at which time the plates were thoroughly washed in PBS with 0.05% Tween (registered Trade Mark) 20, and peroxidase-conjugated goat anti-human IgG (Cappel, 1:1000 dilution) was added. After another two hour incubation the second antibody was removed and substrate added (OPD, Sigma, 100 µl/well). The enzymatic reaction was quenched twenty minutes later by the addition of 50 µl 2N HCl to each well. Absorbances at 490 nm were measured using a Dynatech MR580 plate reader (reference wavelength 405 nm). Plots of the absorbance versus the amount of competing antigen are shown in Figure 2. As shown the guanidine-process IL-2 did not compete significantly.

### Example 2

Example 1 was repeated through the IL-2 refolding step (recovery of supernatant following reduction of guanidine hydrochloride to 0.2 M).

The IL-2 solution (8.8 mg of protein) was acidified with trifluoroacetic acid to a pH of 2.1 and then centrifuged to remove any precipitate. This was loaded on a 1.25 cm by 30 cm column of Vydac C-4 silica equilibrated with 0.1% trifluoroacetic acid in water. The column was eluted with a gradient of acetonitrile containing 0.1% trifluoroacetic acid. The fractions of pure IL-2 were pooled and then dialyzed into 7 M guanidine pH 7.5 buffer. This was then dialyzed against 10 mM phosphate buffer pH 7.0. A precipitate was removed by microcentrifugation to recover 68% of the IL-2 in the supernatant.

### Example 3

Example 1 was repeated through the recovery of the refractile body particle paste.

About 113.7 g of solid guanidine (7 M final concentration) was added to ^{∼}14 g of the particle paste followed by addition of 10 mM Tris/2 mM EDTA buffer to about 190 ml. After homogenizing, ^{∼}1.5 g of solid DTT (50 mM final concentration) was added and the pH was adjusted to 8-8.5 with NaOH. The solution was warmed to 50°C for 15 mins to promote reduction. The solution was then diluted with 10 mM Tris/2 mM EDTA to a final volume of 200 ml.

The next step in the purification was to remove the reducing agent and other contaminants from the IL-2 material. About 25 ml of the reduced IL-2 were diluted to approximately 5-10 mg IL-2/ml with 75 ml 7.0 M guanidine. This solution was diluted to 4.8 M guanidine with Tris/EDTA buffer and allowed to stand two hours. Very little precipitate was formed, and the precipitate was removed by centrifugation at 10,000 x g for 15 minutes. The supernatant was diluted to 4.0 M guanidine with Tris/EDTA buffer and allowed to stand two hours at room temperature. The heavy precipitate was collected by centrifugation (10,000 g x 15 min).

The pellet was washed once with 50 ml 2% Tween 80 and twice with 50 ml H₂O. 16.7 g of solid guanidine was added and the solution was brought to 25 ml with water. The solubilized IL-2 was diluted to 1 mg/ml with 7.0 M guanidine in 10 mM Citrate, pH 6.5. CuCl₂ (to 0.1 mM) was added and the pH was adjusted to 8-8.5. This solution was allowed to stir overnight.

Using a YM spiral-wound cartridge, the guanidine was diafiltered away and the IL-2 was concentrated to about 2 mg/ml. Care was taken to remove air from the system because soluble IL-2 is sensitive to agitation, especially with air bubbles. It was diafiltered against 10 volumes of 2.5% sucrose and 140 mM NaCl in 10 mM NaCitrate pH 6.5 solution. After centrifugation at 3000 g x 15 min, 600 ml of supernatant containing 0.63 mg/ml of IL-2 was collected. The ionic strength was reduced by dialysis into 10 mM NaCitrate, pH 6.5 before proceeding.

The chromatography consisted of two columns, each equilibrated with 10 mM NaCitrate pH 6.5 buffer. The first column was packed with DEAE Sepharose Fast Flow (Pharmacia). 25 ml of 0.63 mg IL-2/ml in 10 mM NaCitrate pH 6.5 was run through a 1 x 10 cm column at 0.5 ml/minute. The pool's NaCl concentration was adjusted to 40 mM NaCl. The second column was packed with CM Sepharose Fast Flow (Pharmacia). 28.5 ml of 0.38 mg IL-2/ml was loaded at 0.5 ml/min. The IL-2 bound to the gel and was eluted with an increasing NaCl gradient (40-400 mM NaCl in 10 mM NaCitrate pH 6.5, 0.3 ml/min) over 6 hours. About 12.6 ml of 0.71 mg of IL-2 per ml was pooled.
The CM pool was desalted into 10 mM sodium citrate pH 6.5 over G25 Sephadex (registered Trade Mark) to remove NaCI and provide a well characterized buffer for formulation.

The resulting IL-2 was over 99% pure by analytical RP-HPLC.

### Example 4

About 5 ml of IL-2-containing refractile bodies, slurried with an equal volume of water was dissolved with guanidine buffer. The solution had a guanidine concentration of 7 M and a volume of 35 ml. The pH of the solution was adjusted to about 8.0 with 3 M Tris base. Next, 0.3 g of DTT was added and the solution was heated to about 45°C for 15 min. The solution was then diluted with an equal volume of 0.1 M citrate pH 5.0 buffer and allowed to stand for 1.5 hours. The formed precipitate was separated by centrifugation (10,000 x g for 10-20 min). The precipitate was washed four times with 70 ml of 3.5 M guanidine and twice with 70 ml of water. The precipitate was dissolved in 7 M guanidine and was analyzed by reverse phase HPLC. Approximately 265 mg of IL-2 were recovered in about 90% purity.

From the foregoing it may be seen that the present process provides advantages as regards: (1) simplicity of the purification process, (2) the absence of solubilizing agent in the final product and (3) recombinant IL-2 product that appears to be less immunogenic than that previously made.

In addition to the aforedescribed vector system employing the trp promoter for IL-2 expression, alternative vector systems include the use of the lambda pL promoter and/or a positive retroregulatory element. These vector systems are described in U.S. Patent Nos. 4,711,845, issued 8 December 1987 and 4,666,848, issued 19 May 1987,

Vector systems described in the aforedescribed patents, as well as additional vectors provided below, have been deposited with the American Type Culture Collection (ATCC), 12301 Parklawn Drive, Rockville, Maryland under the terms of the Budapest Treaty on the International Recognition of the Deposit of Microorganisms for the Purpose of Patent Procedure and Regulations thereunder and are thus maintained and made available according to the terms of the Budapest Treaty. Availability of such strains is not to be construed as a license to practice the invention in contravention of the rights granted under the authority of any government in accordance with its patent laws.

The deposited plasmids have been assigned the indicated ATCC deposit numbers:

| Plasmid | ATCC No. | Deposit Date |
|---|---|---|
| pFC54 in E. coli DG95 lambda (N₇N₅₃cI857SusP₈₀) | 39831 | 4 September 1984 |
| pFC54.t in E. coli DG95 lambda | 39789 | 31 July 1984 |
| pHCW701 in E. coli K12 MM294 | 39757 | 8 June 1984 |
| pLW1 in E. coli K12 MM294 | 39405 | 25 July 1983 |
| pLW46 in E. coli K12 MM294 | 39452 | 29 September 1983 |
| pLW55 in E. coli K12 MM294.1 | 39516 | 29 September 1983 |
| pSY3001 in E. coli K12 MM294 | 39949 | 19 December 1984 |

## Claims

1. A process for recovering recombinant IL-2 from a transformed microorganism containing the IL-2, comprising:
(a) isolating water insoluble IL-2 containing material;
(b) dissolving and denaturing the IL-2 in said water insoluble IL-2 material in the presence of a reducing agent and a strongly denaturing concentration of a chaotropic agent;
(c) reducing the concentration of the chaotropic agent so that the reduced and denatured IL-2 precipitates;
(d) solubilizing the IL-2 precipitate in a sufficient concentration of chaotropic agent to keep the reduced and denatured IL-2 in solution;
(e) oxidizing the solubilized IL-2 while maintaining the concentration of chaotropic agent at a strongly denaturing concentration to form the natural disulfide bridge in IL-2;
(f) reducing the concentration of chaotropic agent to a level at which the oxidized IL-2 is permitted to renaturate and refold into the configuration of native IL-2 and at which insoluble extraneous host proteins precipitate; and
(g) separating and removing said insoluble proteins from the soluble renatured, oxidized IL-2.

2. A process of claim 1, wherein the chaotropic agent is a guanidine salt.

3. A process of claim 2 further comprising washing the IL-2 precipitate of steps (c) in a guanidine solution having a concentration between 2 and 4 M.

4. A process for recovering purified renatured recombinant interleukin-2 (IL-2) from a transformed microorganism containing the lL-2 comprising:
(a) disrupting the cell membrane and cell wall of the microorganism;
(b) separating water insoluble IL-2 containing material from the disruptate;
(c) mixing the insoluble IL-2 containing material of step (b) at a pH of 7 to 9 with an aqueous solution of a reducing agent and a chaotropic agent whereby the IL-2 in the insoluble material is dissolved and denatured;
(d) precipitating the IL-2 out of the IL-2 containing solution and recovering the precipitate;
(e) solubilizing the IL-2 precipitate;
(f) oxidizing the IL-2 in the solution while maintaining the concentration of chaotropic agent at a strongly denaturing concentration, whereby the natural disulfide bridge of IL-2 is formed;
(g) after the oxidation of step (f) is complete, reducing the concentration of chaotropic agent in the solution to a level at which the oxidized IL-2 is permitted to renaturate and a precipitate forms;
(h) separating the precipitate of step (g) from the solution to provide a supernatant;
(i) purifying the oxidized IL-2 in the supernatant by
(1) reverse-phase high performance liquid chromatography followed by dissolution of the pool in solution of chaotropic agent and removal of the chaotropic agent from the solution, or
(2) hydrophobic interaction chromatography combined with ion exchange chromatography, or
(3) ion exchange chromatography; and
(j) recovering a purified oxidized, soluble heterologous human IL-2 composition having an IL-2 content of at least 95% as determined by reducing sodium dodecyl sulfate polyacrylamide gel electrophoresis analysis, a solubility in phosphate buffered saline of at least 5 mg IL-2 per ml, a specific activity of at least 1 x 10⁷ units/mg as determined by HT-2 cell proliferation assay, and an endotoxin content of less than 0.1 nanograms per mg IL-2.

5. A process of claim 4, wherein the chaotropic agent is guanidine hydrochloride and the strongly denaturing concentration is at least 6 M, optionally in step (g) the concentration of guanidine hydrochloride being reduced to below 2 M.

6. A process of claim 5, wherein the strongly denaturing concentration is in the range of 6 to 9 M.

7. A process of any one of claims 4 to 6, wherein in step (d) the IL-2 precipitate is formed by reducing the guanidine hydrochloride concentration to below 5 M, preferably 3 to 4 M, optionally the lL-2 precipitate being collected by centrifugation and washed with a buffer prior to step (e), preferably the buffer comprising 2-4 M guanidine hydrochloride.

8. A process of any one of claims 4 to 7, wherein the oxidation is a controlled oxidation using Cu⁺² ion as an oxidation promoter or o-iodosobenzoic acid as an oxidizing agent.

9. A process of claim 8, wherein the reducing agent is dithiothreitol; in step (d) IL-2 is precipitated by reducing the concentration of guanidine hydrochloride to below 5 M; the oxidation is a controlled oxidation using Cu⁺² ion as an oxidation promoter; in step (g) the concentration of guanidine hydrochloride is reduced to below 0.5 M; and in step (i) the oxidized IL-2 in the supernatant is purified by ion exchange chromatography using a DEAE Sepharose® column and carboxymethyl Sepharose® column or by reverse-phase high performance liquid chromatography or by hydrophobic interaction chromatography using a phenyl agarose column and ion exchange chromatography using a carboxymethyl agarose column.

10. A process of any one of claims 1 to 8, wherein the reducing agent is dithiothreitol.

11. A process of any one of claims 1 to 10, wherein the
IL-2 is des-ala-IL-2ₛₑᵣ₁₂₅.

12. A process of any one of claims 1 to 11, wherein the
IL-2 is substantially free of pyrogens as determined by the U.S.P. rabbit pyrogen test at a dosage of 1.0 x 10³ units per kg.

## Patentansprüche

1. Verfahren zur Gewinnung von rekombinatem IL-2 aus einem transformierten Mikroorganismus enthaltend das IL-2, umfassend:
(a) Isolieren des wasserunlöslichen IL-2 enthaltenden Materials;
(b) Auflösen und Denaturieren des IL-2 in dem wasserunlöslichen IL-2-Material in Gegenwart einer reduzierenden Verbindung und einer start denaturierenden Konzentration einer chaotropen Verbindung;
(c) Vermindern der Konzentration der chaotropen Verbindung, so daß das reduzierte und denaturierte IL-2 präzipitiert;
(d) Lösen des IL-2-Präzipitats in einer ausreichenden Konzentration einer chaotropen Verbindung, um das reduzierte und denaturierte IL-2 in Lösung zu halten;
(e) Oxidieren des gelösten IL-2, unter Aufrechterhaltung der Konzentration der chaotropen Verbindung in einer starken denaturierenden Konzentration, um die natürliche Disulfid-Brücke im IL-2 zu bilden;
(f) Vermindern der Konzentration der chaotropen Verbindung auf einen Wert, der dem oxidierten IL-2 gestattet, zu renaturieren und sich in die Konfiguration von nativem IL-2 zurückzufalten, und bei dem unlösliche fremde Wirtsproteine ausfallen; und
(g) Abtrennen und Entfernen der unlöslichen Proteine von dem löslichen renatunerten, oxidierten IL-2.

2. Verfahren nach Anspruch 1, wobei die chaotrope Verbindung ein Guanidiniumsalz ist.

3. Verfahren nach Anspruch 2, weiter umfassend das Waschen des IL-2-Präzipitats aus Schritt (c) in einer Guanidinlösung mit einer Konzentrationen zwischen 2 und 4 M.

4. Verfahren zur Gewinnung von gereinigtem, renaturiertem rekombinantem Interleukin-2 (IL-2) aus einem transformierten Mikroorganismus enthaltend das IL-2, umfassend:
(a) Zerstören der Zellmembran und der Zellwand des Mikroorganismus;
(b) Abtrennen des wasserunlöslichen IL-2 enthaltenden Materials von dem Aufschluß;
(c) Mischen des unlöslichen IL-2 enthaltenden Materials aus Schritt (b) bei einem pH-Wert von 7 bis 9 mit einer wäßrigen Lösung einer reduzierenden und einer chaotropen Verbindung, wobei das IL-2 in dem unlöslichen Material gelöst und denaturiert wird;
(d) Präzipitieren des IL-2 aus der IL-2 enthaltenden Lösung und Gewinnen des Präzipitats;
(e) Lösen des IL-2-Präzipitats;
(f) Oxidieren des IL-2 in der Lösung unter Aufrechterhaltung der Konzentration der chaotropen Verbindung in einer starken denaturierenden Konzentration, wobei die natürliche Disulfid-Brücke von IL-2 gebildet wird;
(g) nach vollständiger Oxidation in Schritt (f), Vermindern der Konzentration der chaotropen Verbindung in der Lösung auf einen Wert, der dem oxidierten IL-2 gestattet, zu renaturieren und ein Präzipitat zu bilden;
(h) Abtrennen des Präzipitats aus Schritt (g) von der Lösung zur Bereitstellung eines Überstands;
(i) Reinigen des oxidierten IL-2 im Überstand durch
(1) Umkehrphasen-Hochdruck-Flüssigchromatographie gefolgt vom Auflösen des Pools in einer Lösung einer chaotropen Verbindung und Entfernen der chaotropen Verbindung aus der Lösung, oder
(2) hydrophobe Wechselwirkungs-Chromatographie kombiniert mit Ionenaustauschchromatographie, oder
(3) Ionenaustauschchromatographie; und
(j) Gewinnen einer gereinigten, oxidierten, löslichen, heterologen menschlichen IL-2-Zusammensetzung mit einem IL-2-Gehalt von mindestens 95 % bestimmt mit einem reduzierenden Natriumdodecylsulfat-Polyacrylamid-Gelelektrophorese-Nachweis, einer Löslichkeit in phosphatgepufferter Kochsalzlösung von mindestens 5 mg IL-2 pro ml, einer spezifischen Aktivität von mindestens 1 x 10⁷ Einheiten/mg bestimmt mit einem HT-2 Zell-Proliferations-Nachweis und einem Endotoxin-Gehalt von weniger als 0,1 Nanogramm pro mg IL-2.

5. Verfahren nach Anspruch 4, wobei die chaotrope Verbindung Guanidiniumhydrochlorid ist und die stark denaturierende Konzentration mindestens 6 M beträgt, und gegebenenfalls in Schritt (g) die Konzentration von Guanidiniumhydrochlorid unter 2 M vermindert wird.

6. Verfahren nach Anspruch 5, wobei die stark denaturierende Konzentration im Bereich von 6 bis 9 M liegt.

7. Verfahren nach einem der Ansprüche 4 bis 6, wobei in Schritt (d) das IL-2-Präzipitat durch Vermindern der Guanidiniumhydrochlorid-Konzentration unter 5 M, vorzugsweise 3 bis 4 M, gebildet wird, und gegebenenfalls das IL-2-Prazipitat durch Zentrifugation gesammelt und vor Schritt (e) mit einem Puffer gewaschen wird, wobei der Puffer vorzugsweise 2 bis 4 M Guanidiniumhydrochlorid umfäßt.

8. Verfahren nach einem der Ansprüche 4 bis 7, wobei die Oxidation eine kontrollierte Oxidation mit Cu(2+)-Ionen als Oxidationspromotor oder o-Iodosobenzoesäure als Oxidationsmittel ist.

9. Verfahren nach Anspruch 8, wobei die reduzierende Verbindung Dithiothreit ist; wobei in Schritt (d) IL-2 durch Vermindern der Konzentration von Guanidiniumhydrochlorid unter 5 M präzipitiert wird; die Oxidation eine kontrollierte Oxidation mit Cu(2+)-Ionen als Oxidationspromotor ist; in Schritt (g) die Konzentration von Guanidiniumhydrochlorid unter 0,5 M vermindert wird; und in Schritt (i) das oxidierte IL-2 im Überstand durch Ionenaustauschchromatographie mit eher DEAE-Sepharose®-Säule und Carboxymethyl-Sepharose®-Säule oder durch Umkehrphasen-Hochdruck-Flüssigchromatographie oder durch hydrophobe Wechselwirkungs-Chromatographie mit einer Phenylagarose-Säule und Ionenaustauschchromatographie mit einer Carboxymethylagarose-Säule gereinigt wird.

10. Verfahren nach einem der Ansprüche 1 bis 8, wobei die reduzierende Verbindung Dithiothreit ist.

11. Verfahren nach einem der Ansprüche 1 bis 10, wobei das IL-2 Des-Ala-IL-2_{Ser125} ist.

12. Verfahren nach einem der Ansprüche 1 bis 11, wobei das IL-2 im wesentlichen frei von Pyrogenen ist, bestimmt nach dem U.S.P.-Kaninchen-Pyrogen-Test mit einer Dosierung von 1,0 x 10³ Einheiten pro kg.

## Revendications

1. Procédé de récupération d'IL-2 recombinante à partir d'un micro-organisme transformé contenant de l'IL-2, comprenant les étapes consistant à :
(a) isoler le matériau, insoluble dans l'eau contenant l'IL-2 ;
(b) dissoudre et dénaturer l'IL-2 dans ledit matériau, insoluble dans l'eau, contenant l'IL-2 en présence d'un agent réducteur et d'un agent chaotrope à une concentration fortement dénaturante ;
(c) réduire la concentration de l'agent chaotrope de telle sorte que l'IL-2 réduite et dénaturée précipite ;
(d) solubiliser le précipité d'IL-2 avec une concentration suffisante d'agent chaotrope pour maintenir l'IL-2 réduite et dénaturée en solution ;
(e) oxyder l'IL-2 solubilisée en maintenant la concentration d'agent chaotrope au niveau d'une concentration fortement dénaturante pour former les liaisons disulfure naturelles dans IL-2 ;
(f) réduire la concentration d'agent chaotrope à un niveau auquel l'IL-2 oxydée peut se renaturer et se replier dans la configuration de l'IL-2 native, et auquel les protéines étrangères de l'hôte précipitent ; et
(g) séparer et éliminer lesdites protéines insolubles de l'IL-2 soluble oxydée et renaturée.

2. Procédé de la revendication 1, dans lequel l'agent chaotrope est un sel de guanidine.

3. Procédé de la revendication 2 comprenant en outre le lavage du précipité d'IL-2 des étapes (c) dans une solution de guanidine ayant une concentration comprise entre 2 et 4 M.

4. Procédé de récupération d'interleukine-2 (IL-2) recombinante soluble purifiée à partir d'un micro-organisme transformé contenant l'IL-2 comprenant les étapes consistant à :
(a) rompre la membrane cellulaire et la paroi cellulaire du microorganisme;
(b) séparer les substances insolubles dans l'eau, contenant l'IL-2, du produit obtenu par rupture;
(c) mélanger le matériau insoluble de l'étape (b) contenant l'IL-2 à un pH d'environ 7 à environ 9 avec une solution aqueuse d'un agent réducteur et d'un agent chaotrope, ce qui permet la dissolution et la dénaturation de l'IL-2 ;
(d) précipiter l'IL-2 à partir de la solution contenant l'IL-2 et récupérer le précipité ;
(e) solubiliser le précipité d'IL-2 ;
(f) oxyder l'IL-2 dans la solution, en maintenant la concentration d'agent chaotrope à une concentration fortement dénaturante, ce qui permet de former la liaison disulfure naturelle d'IL-2 ;
(g) après achèvement de l'étape d'oxydation (f), réduire la concentration de l'agent chaotrope dans la solution à un niveau auquel l'IL-2 oxydée peut se renaturer et auquel un précipité peut se former ;
(h) séparer le précipité de l'étape (g) de la solution pour obtenir un surnageant ;
(i) purifier l'IL-2 renaturée oxydée dans le surnageant par
(1) chromatographie liquide à haute performance à polarité de phases inversées, puis dissolution de l'ensemble dans une solution d'agent chaotrope et élimination de l'agent chaotrope de la solution, ou
(2) chromatographie d'interaction hydrophobe combinée à une chromatographie d'échange d'ions, ou
(3) chromatographie d'échanges d'ions ; et
(j) récupérer une composition d'IL-2 humaine hétérologue soluble oxydée et purifiée ayant une teneur en IL-2 d'au moins environ 95% selon une analyse par électrophorèse réductrice sur gel de polyacrylamide en présence de dodécylsulfate de sodium, une solubilité d'au moins 5 mg d'IL par ml en solution saline tamponnée de phosphate, une activité specifique d'au moins 1 x 10⁷ unités/mg selon une détermination par un dosage de prolifération de cellules HT-2 et une teneur en endotoxines de moins d'environ 0,1 nanogramme par mg d'IL-2.

5. Procédé de la revendication 4, dans lequel l'agent chaotrope est du chlorhydrate de guanidine et la concentration fortement dénaturante est d'au moins 6 M, la concentration de chlorhydrate de guanidine dans l'étape (g) étant éventuellement ramenée en dessous de 2 M.

6. Procédé de la revendication 5, dans lequel la concentration fortement dénaturante est comprise entre 6 et 9 M.

7. Procédé de l'une quelconque des revendications 4 à 6, dans lequel, dans l'étape (d), on forme le précipité d'IL-2 en réduisant la concentration de chlorhydrate de guanidine en dessous de 5 M, de préférence entre 3 et 4 M, le précipité d'IL-2 étant éventuellement recueilli par centrifugation et lavé avec un tampon avant l'étape (e), le tampon comprenant de préférence 2-4 M de chlorhydrate de guanidine.

8. Procédé de l'une quelconque des revendications 4 à 7, dans lequel l'oxydation est une oxydation contrôlée employant l'ion Cu⁺² comme activateur d'oxydation ou l'acide o-iodosobenzöique comme agent oxydant.

9. Procédé de la revendication 8, dans lequel l'agent réducteur est le dithiothréitol, dans lequel, dans l'étape (d), on précipite l'IL-2 en réduisant la concentration de chlorhydrate de guanidine en dessous de 5 M, dans lequel l'oxydation est une oxydation contrôlée employant l'ion Cu⁺² comme activateur d'oxydation, dans lequel, dans l'étape (g) on réduit la concentration de chlorhydrate de guanidine en dessous de 0,5 M, et dans lequel dans l'étape (i) on purifie l'IL-2 oxydée dans le surnageant par chromatographie d'échange d'ions en utilisant une colonne DEAE-Sepharose® et une colonne carboxyméthyl-Sepharose®, par chromatographie liquide à haute performance à polarité de phases inversées ou par chromatographie d'interaction hydrophobe en utilisant une colonne de phényl-agarose et chromatographie d'échange d'ions en utilisant une colonne de carboxyméthyl-agarose.

10. Procédé de l'une quelconque des revendications 1 à 8, dans lequel l'agent réducteur est le dithiothréitol.

11. Procédé de l'une quelconque des revendications 1 à 10, dans lequel l'IL-2 est des-ala-IL-2ₛₑᵣ₁₂₅.

12. Procédé de l'une quelconque des revendications 1 à 11, dans lequel l'IL-2 est pratiquement dépourvue de pyrogène selon une détermination par le test de pyrogène sur lapin U.S.P. à une dose de 1,0 x 10³ unités par kg.
